(19) Europäisches Patentamt
European Patent Office
Office européen des brevets

(11) **EP 3 393 547 B1**

(12) **EUROPÄISCHE PATENTSCHRIFT**

(45) Veröffentlichungstag und Bekanntmachung des Hinweises auf die Patenterteilung:
**17.11.2021 Patentblatt 2021/46**

(21) Anmeldenummer: **16822904.5**

(22) Anmeldetag: **23.12.2016**

(51) Int Cl.:
*A61M 1/36* (2006.01)     *A61M 1/34* (2006.01)

(86) Internationale Anmeldenummer:
**PCT/EP2016/002181**

(87) Internationale Veröffentlichungsnummer:
**WO 2017/108195 (29.06.2017 Gazette 2017/26)**

(54) **DIALYSEGERÄT MIT MITTELN ZUR ERKENNUNG EINER SHUNT-REZIRKULATION**

DIALYSIS DEVICE WITH MEANS FOR DETECTING A SHUNT RECIRCULATION

UNITÉ DE DIALYSE MUNIE DE MOYENS DE DÉTECTION D'UNE RECIRCULATION PAR SHUNT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2015 DE 102015016854**

(43) Veröffentlichungstag der Anmeldung:
**31.10.2018 Patentblatt 2018/44**

(73) Patentinhaber: **Fresenius Medical Care Deutschland GmbH**
**61352 Bad Homburg (DE)**

(72) Erfinder:
• **KOPPERSCHMIDT, Pascal**
**97456 Dittelbrunn (DE)**
• **GAGEL, Alfred**
**96123 Litzendorf (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
EP-A1- 2 238 996     WO-A1-98/17334
DE-C1- 10 218 846     US-A1- 2014 083 943

**Beschreibung**

Die Erfindung betrifft ein Dialysegerät mit Mitteln zur Erkennung einer Shunt-Rezirkulation.

Werden die arterielle und die venöse Nadel des extrakorporalen Blutkreislaufs eines Dialysegeräts im Bereich eines Shunts (einer zur besseren Durchführbarkeit einer Dialysebehandlung künstlich geschaffenen Kurzschlussverbindung zwischen einer Arterie und einer Vene im Patienten) angelegt, kann eine Shunt-Rezirkulation stattfinden, wobei ein Teil des gereinigten Blutes, das durch die venöse Nadel in das Gefäß des Patienten reinfundiert wird, direkt wieder durch die arterielle Nadel in den extrakorporalen Blutkreislauf gelangt. Eine derartige Shunt-Rezirkulation ist unerwünscht, da die Dialyseeffizienz gesenkt wird. Vor diesem Hintergrund ist es wünschenswert, das Vorhandensein einer Shunt-Rezirkulation detektieren zu können.

Aus der EP 1 790 363 B1 ist ein Dialysegerät bekannt, mit dem eine Detektion einer Shunt-Rezirkulation erfolgen kann. Dieses Gerät weist Hämatokritsensoren in der arteriellen und der venösen Leitung des extrakorporalen Blutkreislaufs auf. Zur Detektion einer Shunt-Rezirkulation ist vorgesehen, durch eine temporäre Erhöhung der Ultrafiltrationsrate am Dialysator einen Peak im Hämatokritwert des extrakorporal geführten Blutes zu erzeugen. Dieser Peak wird dann am venösen Hämatokritsensor erkannt. Wird ein korrespondierender Peak im richtigen zeitlichen Versatz auch am arteriellen Hämatokritsensor erkannt, wird auf eine Shunt-Rezirkulation geschlossen.

US 2014/083943 A1 offenbart eine Vorrichtung zur Erkennung der Rezirkulation für eine extrakorporale Blutbehandlungsvorrichtung sowie eine extrakorporale Blutbehandlungsvorrichtung mit einer Vorrichtung zur Erkennung der Rezirkulation. EP 2 238 996 A1 bezieht sich auf ein Verfahren zur Bestimmung der Fistelrezirkulation für eine Vorrichtung zur extrakorporalen Blutbehandlung mit einem extrakorporalen Blutkreislauf. WO 98/17334 A1 offenbart ein System zur Bestimmung der Rezirkulation von Blut in einem Gefäßzugang. DE 102 18 846 C1 betrifft ein Verfahren zur Unterbrechung oder Fortführung einer extrakorporalen Blutbehandlung mit veränderten Flussraten in einem extrakorporalen Blutkreislauf.

Aufgabe der Erfindung ist es, ein Dialysegerät bereitzustellen, mit dem die Detektion einer Shunt-Rezirkulation in einfacherer Weise erfolgen kann.

Vor diesem Hintergrund betrifft die Erfindung ein Dialysegerät mit einem extrakorporalen Blutkreislauf, der eine arterielle Leitung mit einer Blutpumpe und einer arteriellen Nadel zur Verbindung mit einem Patienten, eine venöse Leitung mit einer venösen Nadel zur Verbindung mit einem Patienten und einen zwischen der arteriellen und der venösen Leitung angeordneten Dialysator mit einer Blutkammer und einer Dialysierflüssigkeitskammer aufweist, wobei das Dialysegerät ferner eine Steuereinheit aufweist. Erfindungsgemäß ist vorgesehen, dass das Dialysegerät ferner einen saugseitig der Blutpumpe angeordneten extrakorporalen Blutdrucksensor aufweist und dass die Steuereinheit so ausgebildet ist, dass eine auf das Vorhandensein einer Rezirkulation hinweisende Signalausgabe erfolgt, wenn eine auf ein Triggerereignis folgende Änderung im Signal des Sensors einen Schwellenwert übersteigt.

Gegenüber dem aus der EP 1 790 363 B1 bekannten System kann somit auf eine komplexere Messung des Hämatokritwertes verzichtet werden und die Bestimmung kann unter einziger Verwendung des ohnehin im Gerät vorhandenen arteriellen Drucksensors erfolgen. Eine Messung in der venösen Leitung bzw. an der Druckseite der Pumpe ist nicht erforderlich.

Erfindungsgemäß handelt es sich bei dem Triggerereignis um eine temporäre Eindickung des druckseitig der Blutpumpe im extrakorporalen Blutkreislauf geführten Blutes. Durch die temporäre Eindickung des Blutes kommt es zu einer temporären Änderung der Blutviskosität. Werden Blutanteile mit einer geänderten Blutviskosität nach Reinfusion an den Patienten im Shunt rezirkuliert und durch die arterielle Nadel erneut in den extrakorporalen Blutkreislauf angesaugt, bewirkt die resultierende temporäre Änderung des Flusswiderstandes an der venösen Nadel eine Änderung des extrakorporalen Blutdrucks auf der Saugseite der Pumpe. Diese Änderung kann durch den Sensor erfasst werden.

In einer Ausführungsform weist das Dialysegerät ferner ein Dilutionssystem mit einer in den extrakorporalen Blutkreislauf mündenden Dilutionsleitung und einer Dilutionspumpe auf, wobei die Steuereinheit so ausgebildet ist, dass das Triggerereignis durch eine Ansteuerung der Dilutionspumpe ausgelöst wird.

In einer Ausführungsform ist die Steuereinheit so ausgebildet, dass das Triggerereignis durch eine temporäre Reduktion der Pumpleistung oder Deaktivierung der Dilutionspumpe ausgelöst wird.

In einer Ausführungsform handelt es sich bei dem Dilutionssystem um ein Postdilutionssystem und die Dilutionsleitung mündet in die venöse Leitung. Bei Verwendung eines Postdilutionssystems kann bei der Detektion einer Shunt-Rezirkulation eine hohe Sensitivität erreicht werden.

Eine temporäre Reduktion der Pumpleistung oder Deaktivierung der Dilutionspumpe führt bei Postdilution zu einer temporären Eindickung des extrakorporalen Blutes. Bei Prädilution stellt sich normalerweise der umgekehrte Effekt ein, was einer Bilanzierung zwischen Substitution und Filtration geschuldet ist. Denn ein Stopp der Prädilution hat normalerweise einen Stopp der Filtration zur Folge, so dass ein verdünnter Bolus resultiert, da das Blut dann durch den Dialysator läuft, ohne filtriert zu werden. Eine temporäre Verdünnung des Blutes kann unabhängig von der Wahl einer Prä- oder Postdilution auch durch eine temporäre Erhöhung der Pumpleistung einer Dilutionspumpe erzielt werden. Dies kann beispielsweise mittels einer zusätzlichen Dilutionspumpe erreicht werden. Durch den nichtlinearen Zusammenhang kann das im Falle einer Rezirkulation am arteriellen Sensor gemessene Signal bei einer Eindickung des Blutes ausgeprägter sein als bei einer Verdünnung des Blutes.

Das erfindungsgemäße Dialysegerät eignet sich in dieser Ausführungsform zum Einsatz im Rahmen einer Hämo(dia)filtrationsbehandlung, wobei die im Dialysator aus dem Blut entfernte Flüssigkeitsmenge durch Pre- oder Postdilution ersetzt wird.

In einer Ausführungsform weist das Dialysegerät ferner eine saugseitig mit der Dialysierflüssigkeitskammer in Verbindung stehende Ultrafiltrationspumpe auf, wobei die Steuereinheit so ausgebildet ist, dass das Triggerereignis durch eine Ansteuerung der Ultrafiltrationspumpe ausgelöst wird. Eine temporäre Reduktion der Pumpleistung der Ultrafiltrationspumpe bewirkt eine temporäre Verringerung des Transmembrandrucks im Dialysator, was zu einer temporären Verringerung des Fluidflusses aus dem Blut und so einer temporären Verdünnung des extrakorporalen Blutes führt. Umgekehrt führt eine temporäre Erhöhung der Pumpleistung führt zu einer temporären Eindickung des Blutes.

In einer Ausführungsform ist die Steuereinheit so ausgebildet, dass das Triggerereignis durch eine temporäre Erhöhung der Pumpleistung der Ultrafiltrationspumpe ausgelöst wird. Wie bereits weiter oben ausgeführt kann durch den nichtlinearen Zusammenhang das im Falle einer Rezirkulation am arteriellen Sensor gemessene Signal bei einer Eindickung des Blutes ausgeprägter sein als bei einer Verdünnung des Blutes.

In diesen Ausführungsformen eignet sich das erfindungsgemäße Dialysegerät zum Einsatz im Rahmen einer Hämodialysebehandlung, in der keine Pre- oder Postdilution stattfindet.

In einer Ausführungsform ist die Steuereinheit so ausgebildet, dass der Signalwert des Sensors unmittelbar vor Eintritt des Triggerereignisses als Ausgangswert gespeichert wird, dass der maximale oder minimale Signalwert des Sensors während einer Messphase nach Eintritt des Triggerereignisses als Peakwert gespeichert wird, und dass die Änderung im Signal der Differenz zwischen Ausgangswert und Peakwert entspricht.

In einer Ausführungsform ist die Steuereinheit so ausgebildet, dass die Dauer der Messphase mindestens der Dauer des Triggerereignisses entspricht und/oder dass die Messphase in zeitlichem Versatz zum Triggerereignis beginnt.

In einer Ausführungsform ist die Steuereinheit so ausgebildet, dass das Integral der Differenz zwischen dem Ausgangswert und den während der Messphase bestimmten Signalwerten bestimmt wird, und dass auf der Grundlage dieses Integrals eine auf das Ausmaß der Rezirkulation hinweisende Signalausgabe erfolgt.

Die Offenbarung betrifft weiterhin ein nicht beanspruchtes Verfahren zur Erkennung einer Shunt-Rezirkulation während einer Dialysebehandlung mit einem erfindungsgemäßen Dialysegerät, wobei eine auf das Vorhandensein einer Rezirkulation hinweisende Signalausgabe erfolgt, wenn eine auf ein Triggerereignis folgende Änderung im Signal des Sensors einen Schwellenwert übersteigt.

Weitere Einzelheiten und Vorteile der Erfindung ergeben sich aus dem nachfolgend anhand der Figuren dargestellten Ausführungsbeispiel. In den Figuren zeigen:

Figur 1:     eine schematische Darstellung eines erfindungsgemäßen Dialysegeräts;

Figur 2:     eine Darstellung der zeitlichen Verläufe arterieller und venöser Drucksignale bei Aussetzung der Postdilution während einer HDF Behandlung bei bestehender Shunt-Rezirkulation; und

Figur 3:     eine Darstellung der fehlenden Korrelation der arteriellen und venösen Drucksignale ohne Shunt-Rezirkulation.

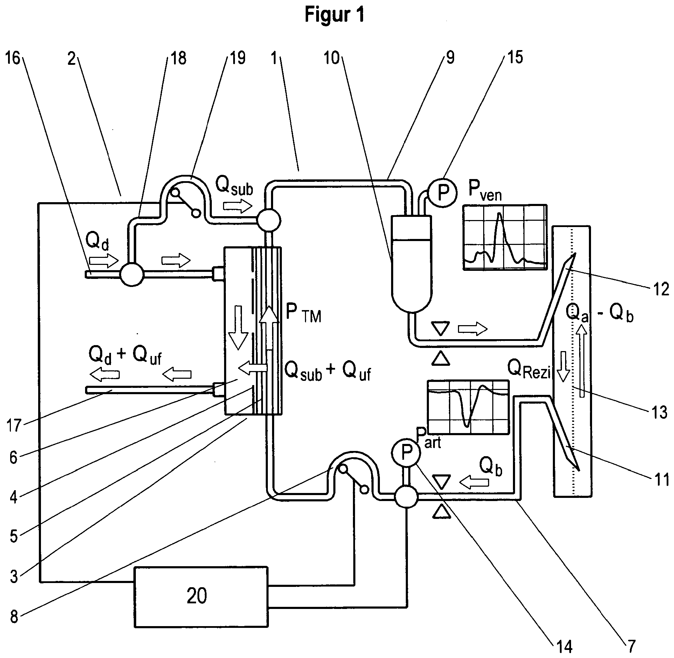

In Figur 1 ist eine erste Ausführungsform eines erfindungsgemäßen Dialysegerätes schematisch dargestellt.

Das Dialysegerät umfasst einen extrakorporalen Blutkreislauf 1 und einen Dialysierflüssigkeitskreislauf 2, welche an einem Dialysator 3 miteinander in Kontakt treten. Der Dialysator 3 umfasst eine semipermeable Membran 4, welche eine Blutkammer 5, die einen Teil des extrakorporalen Blutkreislaufs 1 bildet, und eine Dialysierflüssigkeitskammer 6, die einen Teil des Dialysierflüssigkeitskreislaufs 2 bildet, voneinander trennt. Die Flussrichtungen des Bluts und der Dialysierflüssigkeit in den unterschiedlichen Kammern 5 und 6 des Dialysators 3 sind gegenläufig. Die Flussrichtungen in den Kreisläufen sind in der Figur mit Pfeilen angedeutet.

In der arteriellen Blutleitung 7 befindet sich eine Blutpumpe 8 und in der venösen Blutleitung 9 befindet sich eine Tropfkammer 10. Die arterielle Nadel und die venöse Nadel zum Anschluss des Patienten sind mit den Bezugszeichen 11 und 12 gekennzeichnet. Das Gefäß des Patienten, welches im Bereich eines Shunts liegt, ist mit dem Bezugszeichen 13 gekennzeichnet.

Auf der Saugseite der Blutpumpe 8 befindet sich ein arterieller Drucksensor 14 und an der venösen Tropfkammer 10 befindet sich ein venöser Drucksensor 15.

Die Vorlaufleitung 16 des Dialysierflüssigkeitskreislaufs ist mit einer Dialysierflüssigkeitsquelle verbunden. Die Rücklaufleitung 17 des Dialysierflüssigkeitskreislaufs ist mit einem Ablauf verbunden. Von der Vorlaufleitung 16 zweigt eine Postdilutionsleitung 18 ab, in der eine Dilutionspumpe 19 angeordnet ist. Die Postdilutionsleitung 18 mündet zwischen dem Dialsyator 3 und der Tropfkammer 10 in die venöse Leitung 9.

Das Dialysegerät weist ferner eine Steuereinheit 20 auf, die zumindest mit der Blutpumpe 8, dem arteriellen Drucksensor 14 und der Dilutionspumpe 19 verbunden ist.

Im Rahmen einer an dem gezeigten Gerät durchgeführten post-H(D)F-Behandlung wird das Blut im Dialysator 3 durch die Filtrationsrate $Q_{tm} = Q_{uf} + Q_{sub}$ eingedickt. Vor der venösen Tropfkammer 10 wird das Blut durch den Fluss $Q_{sub}$ der Substituat- bzw. Postdilutionspumpe 19 wieder auf den Wert des Hämatokrits am Dialysatoreingang verdünnt ($Q_{uf}$ ist hier vernachlässigbar).

Sobald die Substituat- bzw. Postdilutionspumpe 19 bei H(D)F-Behandlungen stehen bleibt, läuft ein Hämobolus (vorübergehende zeitliche Änderung des Hämatokrits in Bezug auf stationären Zustand) durch das venöse Schlauchsystem 9. Das eingedickte Blut wird ausgespült und fließt über die venöse Nadel 12 zurück in den Shunt 13 des Patienten. Die sich ändernde Blutviskosität verursacht einen Peak im venösen Druck, der durch den hämodynamischen Druckabfall in der Nadel entsteht.

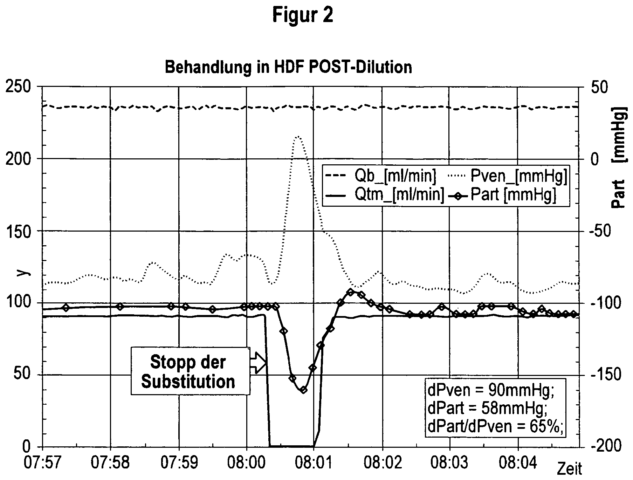

Der zeitliche Verlauf des venösen Drucks wurde im Rahmen einer Beispielmessung ermittelt und ist in Figur 2 dargestellt.

Ein Teil des eingedickten Blutes gelangt über den Rezirkulationsfluss $Q_{Rezi}$ im Shunt 13 zurück zur arteriellen Nadel 11 und wird dort mit Blutfluss $Q_b$ in die arterielle Nadel 11 angesaugt. Durch die Vermischung verändert sich der Hämatokrit des arteriellen Eingangsblutes, wodurch ein Peak zu höheren oder niedrigeren Unterdrücken im arteriellen Druck entsteht. Die Höhe und die Fläche unter dem arteriellen Druckpeak können als Maß für die Rezirkulation dienen.

Die in Figur 2 gezeigten venösen und arteriellen Druckpeaks entstehen durch dynamische Druckabfälle in der jeweiligen Nadel 11 bzw. 12, die durch den Fluss des eingedickten Blutes verursacht werden. Da das Blutvolumen im Shunt 13 sehr gering ist, treten die beiden Peaks praktisch gleichzeitig auf. Die Kurvenform der beiden spiegelverkehrten Signale ist ähnlich (ohne Rauschen) zueinander. Daher sind sowohl die Koinzidenz der beiden Peaks als auch die Ähnlichkeit der Kurvenformen der beiden Drucksignale hilfreiche Kriterien zur Erkennung einer Rezirkulation.

Die Bewertung der Signale durch die in der Steuereinheit 20 hinterlegten Routine, welche dazu dient, eine erhöhte Shunt-Rezirkulation zu erkennen, kann in der gezeigten Ausführungsform beispielsweise wie folgt erfolgen.

Der arterielle Druck $P_{art,Start}$ unmittelbar vor dem Triggerzeitpunkt kann gespeichert werden. Nach dem Triggerzeitpunkt kann mit Hilfe des aktuellen arteriellen Druckes $P_{art}$ die folgende Differenz des arteriellen Drucks $\Delta P_{art}$ berechnet werden:

$$\Delta P_{art}(t) = P_{art}(t) - P_{art,Start} \tag{1}$$

Anschließend kann der Peak in der Druckdifferenz wie folgt bestimmt werden, wobei der Triggerzeitpunkt der Beginn des Ablaufs mit $t = 0$, $V_b = 0$ und $\Delta P_{art,Peak} = 0$ ist:

$$\Delta P_{art,Peak,neu} = \begin{cases} P_{art}(t) - P_{art,Start} & \textit{falls } \Delta P_{art,Peak,alt} > P_{art}(t) - P_{art,Start} \textit{ bei Eindickung} \\ \Delta P_{art,Peak,alt} & \textit{sonst} \\ P_{art}(t) - P_{art,Start} & \textit{falls } \Delta P_{art,Peak,alt} < P_{art}(t) - P_{art,Start} \textit{ bei Verdünnung} \end{cases} \tag{2}$$

Die Peaksuche kann dabei auf ein Zeit-(t-) oder ein gefördertes Blutvolumen-($V_b$-) Intervall begrenzt werden, in dem der erwartete Peak auftreten muss.

Vorzugsweise wird dabei in der folgenden Art zum einen eine Verzögerung beachtet, die das Blut benötigt, um vom Dialysator bis zur venösen Nadel zu gelangen:

$$t_{delay} = V_{SS,ven} / Q_b \tag{3}$$

Dabei stellt $t_{delay}$ die Verzögerungszeit gemessen beispielsweise in Minuten dar, die verstreicht, bis der Peak auftreten kann. $V_{SS,ven}$ stellt das Verzögerungsvolumen gemessen beispielsweise in mL dar, welches beispielsweise mit dem Volumen des venösen Schlauchsystems gleichgesetzt werden kann. Letztlich stellt $Q_b$ den Blutfluss dar, ausgedrückt beispielsweise in mL/min.

Vorzugsweise wird dabei zum anderen in der folgenden Art und Weise eine aktive Dauer beachtet, in der Peak auftreten muss.

$$t_{Peak} = \alpha * V_{dialyzer} / Q_b \tag{3}$$

Dabei stellt $t_{Peak}$ das beispielsweise in Minuten ausgedrückte Zeitintervall dar, in dem der Peak auftreten muss. Ferner stellt $V_{dialyzer}$ das geförderte Blutvolumen dar, in dem der Peak auftreten muss. Dies kann beispielsweise mit dem Volumen der Blutkammer 5 des Dialysators 3 gleichgesetzt werden und in mL ausgedrückt werden. Die Variable a beschreibt einen zusätzlichen Gewichtungsfaktor, der berücksichtigt, dass bei einer post-H(D)F-Behandlung das Blut mit der höchsten Eindickung am Dialysator-Ausgang steht, während bei pre-H(D)F das Blut mit der höchsten Verdünnung am Dialysator-Eingang steht. Zum Beispiel können bei einer typischen post-H(D)F das Verzögerungsvolumen in der Größenordnung von 55 mL und das Blutvolumen in der aktiven Dauer in der Größenordnung von 65 mL liegen.

In weiterer Folge kann verlangt sein, dass der Peak in der Druckdifferenz einen Schwellwert $\Delta P_{art,Peak,Limit}$ überschreitet, damit das Vorhandensein einer erhöhten Rezirkulation angenommen werden kann. Der Schwellwert $\Delta P_{art,Peak,Limit}$ kann beispielsweise in Abhängigkeit der Filtrationsfraktion $FF = (Q_{uf}+Q_{sub})/Q_b$ definiert werden, weil mit zunehmender Filtrationsfraktion FF auch die Eindickung oder Verdünnung des Blutes zunimmt.

Zum Beispiel wird in Gleichung (4) ein bei einer post-H(D)F Behandlung vorteilhafter Zusammenhang aufgezeigt.

$$\Delta P_{art,Peak,Limit} = 10\,\text{mmHg} * \left( 1 + \frac{FF - 15\%}{35\%} \right) \text{ für } FF \geq 15\% \qquad (4)$$

In diesem Fall würde bei FF < 15% keine Auswertung vorgenommen.

Anschließend kann eine Überprüfung der Peakform im arteriellen Druck vorgenommen werden. Beispielsweise kann, nachdem die maximale Peakhöhe $\Delta P_{art,Peak}$ detektiert worden ist, erwartet werden, dass die Druckdifferenz $\Delta P_{art}$ (t) wieder gegen Null strebt oder zumindest eine bestimmte Schwelle wieder unterschreitet.

Ferner kann im Zuge des erfindungsgemäßen Konzepts die Schwankungsbreite $\sigma_{Part}$ des ungestörten arteriellen Drucks berücksichtigt werden. Beispielsweise wird angenommen, dass die Peakhöhe $\Delta P_{art,Peak}$ deutlich größer sein muss als die Schwankungsbreite $\sigma_{Part}$ des ungestörten arteriellen Druckes $P_{art}$:

$$\left| \frac{\Delta P_{art,Peak}}{\sigma_{art}} \right| \geq k \qquad (5)$$

In einer Ausgestaltung kann dabei verlangt werden, dass bei post-H(D)F das Verhältnis k > 3,0 . . 4,0 betragen muss.

Gegebenenfalls kann vorgesehen sein, dass, wenn einer Messung eine erhöhte Rezirkulation erkannt worden ist, diese wiederholt wird, um das erste Ergebnis zu verifizieren. Wenn beide Ergebnisse nur schwach positiv waren, kann des Weiteren auch mit einer dritten oder mit weiteren Messungen versucht werden, das Ergebnis zu bestätigen.

Des Weiteren kann bei der Bewertung gegebenenfalls auch eine von OCM (Online Clearance Measurement) gemessene Clearance $Cl_{OCM}$ berücksichtigt werden. Wenn die Clearance $Cl_{OCM}$ oder besser die Verhältnisse $Cl_{OCM}/Q_b$ oder $Cl_{OCM}*t/V_b$ signifikant niedriger als ein üblicher Schwellwert liegen, kann das in einer Ausgestaltung ebenfalls als Hinweis auf eine hohe Shunt-Rezirkulation herangezogen und bei der Bewertung des Ergebnisses des arteriellen Druckpeaks mit berücksichtigt werden.

Gegebenenfalls kann eine Warnung für den Anwender, dass eine erhöhte Shunt-Rezirkulation vorliegt, unmittelbar nach der Auswertung der Ergebnisse erfolgen. Alternativ kann die Meldungsausgabe auch später, zum Beispiel nach der Quittierung der Meldungsausgabe erfolgen, dass das Behandlungsziel erreicht worden ist.

Die erfinderische Idee enthält nicht notwendigerweise den Anspruch, den Rezirkulationsfluss direkt zu messen. Ziel ist es vielmehr eine ungewöhnlich hohe Rezirkulation zu erkennen und dem Anwender zu melden. Damit können nachgelagerte Untersuchungen getriggert werden, bei denen z. B. der Rezirkulationsfluss mit Hilfe eines dezidierten Verfahrens genau bestimmt werden kann.

Die Methode ist besonders sensitiv bei post-H(D)F-Behandlungen in einem Gerät gemäß Figur 1. Aufgrund des nichtlinearen Zusammenhangs wird die Blutviskosität durch Eindickung des Blutes in der Regel stärker verändert als durch die Verdünnung bei pre-H(D)F.

Allerdings ist in einer alternativen Ausführungsform auch die Anwendung im Rahmen einer pre-H(D)F Behandlung denkbar. In diesem Fall wird das Blut stromauf vom Dialysator durch die Substitutionsrate $Q_{sub}$ verdünnt. Das Blut wird im Dialysator durch die Filtrationsrate $Q_{tm} = Q_{uf} + Q_{sub}$ wieder eingedickt, so dass der Hämatokrit am Dialysatorausgang wieder dem ursprünglichen Wert entspricht ($Q_{uf}$ ist hier vernachlässigbar).

Für diese im Rahmen der Erfindung angedachten Maßnahmen sind vorzugsweise keine zusätzlichen Hardware-Komponenten im Dialysegerät erforderlich und die standardmäßig vorhandenen Sensoren und Aktoren ausreichend.

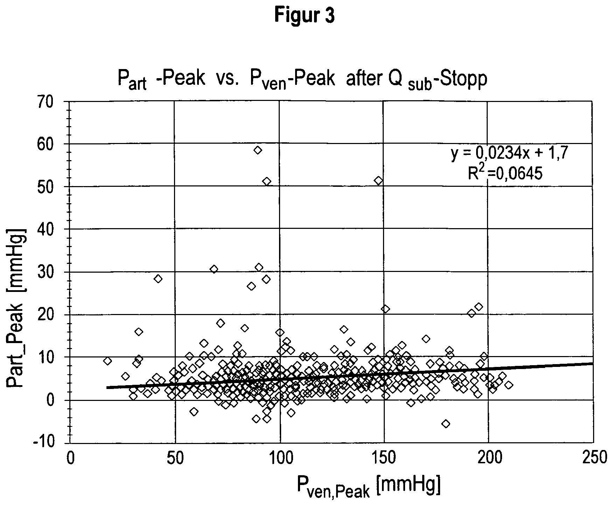

Eine Bestimmung der Höhe bzw. der Integralfläche des venösen Druckpeaks ist im Rahmen der Erfindung prinzipiell nicht erforderlich, weil die Höhe des Peaks im arteriellen Druck nicht von der Peakhöhe des venösen Drucks abhängt, solange keine Rezirkulation vorhanden ist. Vergleiche in diesem Zusammenhang die Darstellung der Figur 3, worin Peakhöhen im arteriellen Druck gegen Peakhöhen im venösen Druck aufgetragen sind, die durch Hämoboli infolge von Stopps der Substitutionspumpe bei post-HDF-Behandlungen (N=967) verursacht wurden. Nur wenn die Höhe des arteriellen Peaks außerhalb der Streubandbreite liegt, kann vermutet werden, dass eine erhöhte Rezirkulation vorliegt.

Die Erfindung ist primär bei pre- und post-H(D)F-Behandlungen anwendbar. Bei HD-Behandlungen kann die Eindickung des Blutes z. B. durch eine kurzzeitige hohe UF-Rate $Q_{uf}$ erzeugt werden.

Als Bedingung zur Auslösung des Triggerereignisses kann eine systembedingte signifikante Änderung der Substitutionsrate herangezogen werden. Eine besonders hohe Änderung stellt beispielsweise Stopp oder Wiederanlauf der Substitutionspumpe dar, was in jeder Behandlung beispielsweise durch einen event-gesteuerten Druckhaltetest erzwungen werden kann.

Zusammenfassend ergibt sich, dass die gezeigte Ausführungsform der Erfindung ein Dialysegerät mit der Fähigkeit bereitstellt, im Rahmen einer post-HDF Behandlung eine Rezirkulation im Shunt zu erkennen. Durch Abschalten der (Prä- oder Post-) Substitutionspumpe wird ein Hämobolus im extrakorporalen Blutkreislauf erzeugt, da die Ultrafiltrationspumpe weiterhin das Blut eindickt. Dieser Bolus erzeugt zunächst auf der venösen Seite einen Druckpeak. Liegt nun eine Fistel-Rezirkulation vor, wandert der Bolus fast sofort durch den Patienten auf die arterielle Seite des extrakorporalen Blutkreislaufs. Durch die Gleichzeitigkeit der Peaks wird auf Rezirkulation geschlossen. Gegenüber der EP 1 790 363 B1 zielt die vorliegende Idee darauf ab, die Rezirkulation vollständig ohne die Bestimmung des venösen Drucks zu erkennen. Dazu wird der arterielle Peak in zeitliche Relation zu dem Ereignis gesetzt, das den venösen Peak verursacht, also insbesondere das Abschalten der Substitutionspumpe. Außerdem wird die Rezirkulation nicht notwendigerweise in ihrer Höhe bestimmt. Stattdessen kann auch lediglich, insbesondere bei sehr hoher Rezirkulation, aufgrund des arteriellen Peaks auf die Tatsache einer Rezirkulation geschlossen werden.

**Patentansprüche**

1. Dialysegerät mit einem extrakorporalen Blutkreislauf (1), der eine arterielle Leitung (7) mit einer Blutpumpe (8) und einer arteriellen Nadel (11) zur Verbindung mit einem Patienten, eine venöse Leitung (9) mit einer venösen Nadel (12) zur Verbindung mit einem Patienten und einen zwischen der arteriellen und der venösen Leitung (7, 9) angeordneten Dialysator (3) mit einer Blutkammer (5) und einer Dialysierflüssigkeitskammer (6) aufweist, wobei das Dialysegerät ferner eine Steuereinheit (20) aufweist, wobei das Dialysegerät ferner einen saugseitig der Blutpumpe (8) angeordneten extrakorporalen Blutdrucksensor (14) aufweist und dass die Steuereinheit (20) so ausgebildet ist, dass eine auf das Vorhandensein einer Rezirkulation hinweisende Signalausgabe erfolgt, wenn eine auf ein Triggerereignis folgende Änderung im Signal des Sensors (14) einen Schwellenwert übersteigt,

   **dadurch gekennzeichnet,**
   **dass** es sich bei dem Triggerereignis um eine temporäre Eindickung des druckseitig der Blutpumpe (8) im extrakorporalen Blutkreislauf (1) geführten Blutes handelt.

2. Dialysegerät nach Anspruch 1, **dadurch gekennzeichnet, dass** es ferner ein Dilutionssystem mit einer in den extrakorporalen Blutkreislauf (1) mündenden Dilutionsleitung (18) und einer Dilutionspumpe (19) aufweist, wobei die Steuereinheit (20) so ausgebildet ist, dass das Triggerereignis durch eine temporäre Reduktion der Pumpleistung oder Deaktivierung der Dilutionspumpe (19) ausgelöst wird.

3. Dialysegerät nach Anspruch 2, **dadurch gekennzeichnet, dass** es sich bei dem Dilutionssystem um ein Postdilutionssystem handelt und die Dilutionsleitung (18) in die venöse Leitung (9) mündet.

4. Dialysegerät nach einem der vorhergehenden Ansprüche, **dadurch gekennzeichnet, dass** es ferner eine saugseitig mit der Dialysierflüssigkeitskammer (6) in Verbindung stehende Ultrafiltrationspumpe aufweist, wobei die Steuereinheit (20) so ausgebildet ist, dass das Triggerereignis durch eine temporäre Erhöhung der Pumpleistung der Ultrafiltrationspumpe ausgelöst wird.

5. Dialysegerät nach einem der vorhergehenden Ansprüche, **dadurch gekennzeichnet, dass** die Steuereinheit (20) so ausgebildet ist, dass der Signalwert des Sensors (14) unmittelbar vor Eintritt des Triggerereignisses als Ausgangswert gespeichert wird, dass der minimale Signalwert des Sensors (14) während einer Messphase nach Eintritt des Triggerereignisses als Peakwert gespeichert wird, und dass die Änderung im Signal der Differenz zwischen Ausgangswert und Peakwert entspricht.

6. Dialysegerät nach Anspruch 5, **dadurch gekennzeichnet, dass** die Steuereinheit (20) so ausgebildet ist, dass die Dauer der Messphase mindestens der Dauer des Triggerereignisses entspricht und/oder dass die Messphase in zeitlichem Versatz zum Triggerereignis beginnt.

7. Dialysegerät nach Anspruch 5 oder 6, **dadurch gekennzeichnet, dass** die Steuereinheit (20) so ausgebildet ist, dass das Integral der Differenz zwischen dem Ausgangswert und den während der Messphase bestimmten Signalwerten bestimmt wird, und dass auf der Grundlage dieses Integrals eine auf das Ausmaß der Rezirkulation hinweisende Signalausgabe erfolgt.

**Claims**

1. Dialysis apparatus with an extracorporeal blood circuit (1), which has an arterial line (7) with a blood pump (8) and an arterial needle (11) for connection to a patient, a venous line (9) with a venous needle (12) for connection to a patient and a dialyzer (3) arranged between the arterial and the venous line (7, 9) with a blood chamber (5) and a dialysis liquid chamber (6), wherein the dialysis apparatus further comprises a control unit (20), wherein the dialysis apparatus further comprises an extracorporeal blood pressure sensor (14) arranged on the suction side of the blood pump (8) and the control unit (20) is formed so that a signal is output indicating the presence of recirculation when a change in the signal of the sensor (14) following a trigger event exceeds a threshold value,

   **characterized in that**
   the trigger event is a temporary thickening of the blood carried on the pressure side of the blood pump (8) in the extracorporeal blood circuit (1).

2. Dialysis apparatus according to claim 1, **characterized in that** it further comprises a dilution system with a dilution line (18) flowing into the extracorporeal blood circuit (1) and a dilution pump (19), wherein the control unit (20) is formed so that the trigger event is triggered by a temporary reduction in the pumping capacity or deactivation of the dilution pump (19).

3. Dialysis apparatus according to claim 2, **characterized in that** the dilution system is a post-dilution system and the dilution line (18) flows into the venous line (9).

4. Dialysis apparatus according to any one of the previous claims, **characterized in that** it further comprises an ultrafiltration pump connected on the suction side to the dialysis liquid chamber (6), wherein the control unit (20) is formed so that the trigger event is triggered by a temporary increase in the pumping capacity of the ultrafiltration pump.

5. Dialysis apparatus according to any one of the previous claims, **characterized in that** the control unit (20) is formed so that the signal value of the sensor (14) directly before the occurrence of the trigger event is stored as an initial value, the minimal signal value of the sensor (14) during a measuring phase following the occurrence of the trigger event is stored as a peak value, and the change in the signal corresponds to the difference between initial value and peak value.

6. Dialysis apparatus according to claim 5, **characterized in that** the control unit (20) is formed so that the duration of the measuring phase corresponds at least to the duration of the trigger event and/or that the measuring phase begins offset in time to the trigger event.

7. Dialysis apparatus according to claim 5 or 6, **characterized in that** the control unit (20) is formed so that the integral of the difference between the initial value and the signal values determined during the measuring phase is determined, and that on the basis of this integral a signal is output indicating the extent of the recirculation.

**Revendications**

1. Appareil de dialyse avec un circuit de sang extracorporel (1), qui présente un conduit artériel (7) avec une pompe à sang (8) et une aiguille artérielle (11) destinée à être reliée à un patient, un conduit veineux (9) avec une aiguille veineuse (12) destinée à être reliée à un patient et un dialyseur (3) disposé entre le conduit artériel et le conduit veineux (7, 9) avec une chambre de sang (5) et une chambre de liquide de dialyse (6), dans lequel l'appareil de dialyse présente en outre une unité de commande (20), dans lequel l'appareil de dialyse présente en outre un capteur de pression artérielle (14) extracorporel disposé côté aspiration de la pompe à sang (8), et l'unité de commande (20) est configurée de telle sorte qu'une émission de signal soulignant la présence d'une recirculation est effectué quand une modification suivant un événement déclencheur dans le signal du capteur (14) dépasse une valeur de seuil,

>**caractérisé en ce que** l'événement déclencheur est un épaississement temporaire du sang guidé côté pression de la pompe à sang (8) dans le circuit de sang extracorporel (1).

2. Appareil de dialyse selon la revendication 1, **caractérisé en ce qu'**il présente en outre un système de dilution avec un conduit de dilution (18) débouchant dans le circuit de sang extracorporel (1) et une pompe de dilution (19), dans lequel l'unité de commande (20) est configurée de telle sorte que l'événement déclencheur est déclenché par une réduction temporaire de la puissance de pompe ou par une désactivation de la pompe de dilution (19).

3. Appareil de dialyse selon la revendication 2, **caractérisé en ce que** le système de dilution est un système de postdilution et le conduit de dilution (18) débouche dans le conduit veineux (9).

4. Appareil de dialyse selon l'une quelconque des revendications précédentes, **caractérisé en ce qu'**il présente en outre une pompe d'ultrafiltration reliée côté aspiration à la chambre de liquide de dialyse (6), dans lequel l'unité de commande (20) est configurée de telle sorte que l'événement déclencheur est déclenché par une augmentation temporaire de la puissance de pompe de la pompe d'ultrafiltration.

5. Appareil de dialyse selon l'une quelconque des revendications précédentes, **caractérisé en ce que** l'unité de commande (20) est configurée de telle sorte que la valeur de signal du capteur (14) est mémorisée directement avant la survenue de l'événement déclencheur en tant que valeur de départ, que la valeur de signal minimale du capteur (14) est mémorisée pendant une phase de mesure après la survenue de l'événement déclencheur en tant que valeur de pic, et que la modification dans le signal correspond à la différence entre la valeur de départ et la valeur de pic.

6. Appareil de dialyse selon la revendication 5, **caractérisé en ce que** l'unité de commande (20) est configurée de telle sorte que la durée de la phase de mesure correspond au moins à la durée de l'événement déclencheur, et/ou que la phase de mesure commence avec un décalage temporel par rapport à l'événement déclencheur.

7. Appareil de dialyse selon la revendication 5 ou 6, **caractérisé en ce que** l'unité de commande (20) est configurée de telle sorte que l'intégrale de la différence entre la valeur de départ et les valeurs de signal déterminés pendant la phase de mesure est définie, et qu'une émission de signal soulignant l'ampleur de la recirculation est effectué sur la base de ladite intégrale.

## Figur 1

## Figur 2

### Behandlung in HDF POST-Dilution

Legend:
- --- Qb_[ml/min]
- ······ Pven_[mmHg]
- —— Qtm_[ml/min]
- —◇— Part [mmHg]

Stopp der Substitution

dPven = 90mmHg;
dPart = 58mmHg;
dPart/dPven = 65%;

## Figur 3

$P_{art}$ -Peak vs. $P_{ven}$-Peak after $Q_{sub}$-Stopp

$y = 0,0234x + 1,7$
$R^2 = 0,0645$

Part_Peak [mmHg]

$P_{ven,Peak}$ [mmHg]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1790363 B1
- US 2014083943 A1
- EP 2238996 A1
- WO 9817334 A1
- DE 10218846 C1